# EUROPÄISCHE PATENTSCHRIFT

(11) **EP 1 222 903 B1**
(45) Veröffentlichungstag und Bekanntmachung des Hinweises auf die Patenterteilung: **19.01.2005**
(21) Anmeldenummer: 01125792.0
(22) Anmeldetag: 29.10.2001
(51) Int. Cl.: A61F 2/46

(54) **Chirurgisches Instrument zum Einsetzen einer Zwischenwirbelendoprothese**
Surgical instrument for implanting an intervertebral prosthesis
Instrument chirurgical pour implanter une prothèse intervertébrale

(30) Priorität: 12.01.2001 EP 01100754
(43) Veröffentlichungstag der Anmeldung: 17.07.2002
(73) Patentinhaber: Link Spine Group, Inc., Branford, CT 06405 (US)
(72) Erfinder: Keller, Arnold, 23863 Kayhude (DE)
(74) Vertreter: Glawe, Delfs, Moll & Partner

(56) Entgegenhaltungen:
- EP-A- 0 077 159
- EP-A- 0 333 990
- DE-U- 20 012 549
- DE-U- 29 916 078
- FR-A- 2 737 656
- US-A- 3 486 505
- US-A- 5 431 658

## Beschreibung

Zum Einsetzen einer Zwischenwirbel-Endoprothese, die aus zwei jeweils mit einem Wirbel zu verbindenden Prothesenplatten und einem Prothesenkern besteht, ist ein Instrument bekannt (EP-B-333990), das zwei gabelförmige Prothesenhalterungen für die zwei Platten aufweist. Diese Führungen sind an einer Spreizzange angeordnet, die es erlaubt, zunächst nur die einander angenäherten Platten ohne den Prothesenkern zwischen zwei Wirbel einzusetzen, sie dann durch Spreizen der Zange auseinanderzuführen, damit der Prothesenkern zwischen die Platten eingesetzt werden kann, und sie schließlich wieder bis zum endgültigen Einschluß des Prothesenkerns einander anzunähern. Nachdem die Prothese so die ihr zugedachte Position erreicht hat, kann das Instrument abgenommen werden. Die gabelförmigen Prothesenhalterungen sind am Ende offen ausgebildet, so daß das Instrument einfach abgezogen werden kann.

Beim Abziehen des Instruments von der Prothese ergeben sich jedoch mitunter Schwierigkeiten durch hohe Reibkräfte, die durch Verkanten des Instruments oder durch Verklemmung der Prothesenhalterungen an den Wirbeln hervorgerufen sein können. Es besteht dann die Gefahr, daß die Prothese sich aufgrund dieser Reibung oder aufgrund heftiger Instrumentenbewegung unerwünscht verschiebt.

Bei einem gattungsfremden Instrument (DE-U-200 12 549; US-A-5 431 658), das zum Einführen eines einstückigen Implantats zwischen zwei Wirbel bestimmt ist, ist es bekannt, das Implantat zwischen einem Paar von Führungsstangen mittels einer Vorschubstange einzuschieben, die einen wirbelanschlag aufweist. Wenn das Implantat die vorgesehene Tiefe im Wirbelzwischenraum erreicht hat, legt sich dieser Anschlag an einem der Wirbel an, wodurch bei weiterer Vorschubbewegung das Instrument von den Wirbeln und der Prothese abgedrückt wird. Diese Lösung ist bei solchen Instrumenten (DE-U-299 16 078) nicht ohne weiteres anwendbar, bei denen ebenso wie bei der erfindungsgemäß vorausgesetzten Gattung zwei Halterungen für Prothesenplatten vorgesehen sind, die zum Einfügen eines Prothesenkerns auseinandergespreizt werden müssen.

Der Erfindung liegt die Aufgabe zugrunde, das Abziehen des eingangs genannten Instruments zu erleichtern. Sie erreicht dies durch die Merkmale des Anspruchs 1.

Demnach ist als Abdrückeinrichtung an jeder Prothesenhalterung ein Schieber angeordnet, der einen Prothesenanschlag und/oder einen Wirbelanschlag aufweist und in einer Schieberführung zwischen einer zurückgezogenen und einer vorgeschobenen Endstellung verschiebbar ist. Dazu dient eine Betätigungseinrichtung, die eine vom gegenseitigen Abstand der Prothesenplatten unabhängige Einrichtung zur Kopplung der Bewegung beider Schieber umfaßt.

Der Schieber, der den Prothesen- bzw. Wirbelanschlag trägt, ist vorzugsweise in einer zur Führungsrichtung der Prothesenhalterung parallelen Schieberführung an dem Instrument beweglich. Er kann in dieser Führung eine hintere Endstellung einnehmen, in der die Prothese vollständig von der Prothesenhalterung erfaßt ist. In dieser Endstellung befindet sich der Schieber, bis die Prothese die ihr zugedachte Implantationsstellung erreicht hat. In der vorderen Endstellung des Schiebers ist der Prothesen- bzw. Wirbelanschlag so weit vorgeschoben, daß die Prothese von der Prothesenhalterung nicht mehr gehalten wird und das Instrument ohne weiteres abgenommen werden kann. Wenn die Prothese eingesetzt ist und die Abdrückeinrichtung betätigt wird, wird das Instrument durch den Weg des sich an der Prothese bzw. am Wirbel abstützenden Schiebers im Verhältnis zu diesen Teilen zurückbewegt, d.h. davon abgedrückt.

Dieses Abdrücken verlangt einen gewissen Kraftaufwand. Zweckmäßigerweise ist deshalb eine Übersetzungseinrichtung zwischen der Abdrückeinrichtung und einer zu deren Betätigung vorgesehenen Handhabe angeordnet. Diese Übersetzungseinrichtung kann beispielsweise von einer Gewindespindel gebildet sein, die an einem Ende auf den Schieber wirkt und am anderen Ende einen vom Arzt zu betätigenden Drehknopf trägt. In einer bevorzugten Ausführungsform wird die Übersetzungseinrichtung von einer Hebeleinrichtung gebildet.

Die Handhabe und die Übersetzung können permanent an dem Instrument angeordnet sein. Um aber die Handhabung des Instruments zu erleichtern und die Übersicht während des Einsetzvorgangs nicht durch unnötige Teile zu beeinträchtigen, kann es zweckmäßig sein, die Handhabe (gegebenenfalls mit der Übersetzungseinrichtung) gesondert von dem Instrument vorzusehen und diese Teile so auszubilden, daß sie bei in Operationsstellung befindlichem Instrument leicht mit diesem verbunden werden können. Beispielsweise werden die Handhabe und die Übersetzungseinrichtung von einer Zange gebildet. Diese weist an einem ihrer kurzen Hebel (Arbeitshebel) Kupplungsmittel auf, die zu Kupplungsmitteln am Instrument komplementär ausgebildet sind. Der andere Arbeitshebel der Zange gelangt mit dem Schieber bzw. mit den Schiebern in Eingriff, wenn die Zange mit dem Instrument verbunden wird.

Beim Abdrücken sollte die Abdrückkraft auf jeden der beiden Schieber wirken, damit nicht eine Prothesenplatte stärker als die andere belastet wird. Das gilt unabhängig von dem gegenseitigen Abstand der Platten. Um dies zu ermöglichen, ist erfindungsgemäß zwischen der Abdrückeinrichtung und den damit zusammenwirkenden Teilen der Schieber eine Wippe angeordnet, über deren Schwenkpunkt die Abdrückkraft im wesentlichen zu gleichen Teilen auf beide Schieber übertragen wird.

Bei einer anderen Ausführungsform der Erfindung ist die Betätigungseinrichtung fest mit dem Instrument verbunden, und zwar mit einem Instrumentenkörper, der lediglich mit einer der beiden Prothesenhalterungen fest verbunden ist. Demzufolge wirkt die Betätigungseinrichtung auch nur auf diesen einen Schieber unmittelbar. Zum synchronen Antrieb des anderen Schiebers ist mit dem unmittelbar angetriebenen Schieber ein Treiberteil bewegungsverbunden, der über eine abstandsunabhängige Kupplung mit einem getriebenen Teil des anderen Schiebers in Eingriff steht. Vorzugsweise wird dies dadurch verwirklicht, daß mit dem direkt angetriebenen Schieber ein Anschlag verbunden ist, der mit ihm in Abdrückrichtung geführt ist und unabhängig vom gegenseitigen Abstand der Prothesenführungen in den Bewegungsbereich eines anderen, mit dem anderen Schieber verbundenen Anschlags reicht. Es sind aber auch andere Lösungen denkbar. Beispielsweise kann der unmittelbar angetriebene Schieber eine Zahnstange tragen, deren Bewegung ein Ritzel antreibt, das mit einer Keilwelle fest verbunden ist, die im Bereich des anderen Schiebers ein auf ihr axial verschiebbares Ritzel trägt, das seine Bewegung auf eine mit dem anderen Schieber verbundene Zahnstange überträgt.

Zu einem besonders einfachen und leicht bedienbaren Instrument kommt man dann, wenn man die Betätigungseinrichtung nicht abnehmbar macht, sondern beispielsweise als Schubstange in den Instrumentenkörper integriert, wobei mit dem Ende der Schubstange ein Griff verbunden sein kann, der in Längsrichtung des Geräts verschoben wird.

Wenn kombinierte Wirbel- und Prothesenanschläge vorhanden sind, können diese dazu verwendet werden, die Position der Prothese zwischen den Wirbeln zu bestimmen, indem das Instrument so weit eingeführt wird, bis der Wirbelanschlag oder die Wirbelanschläge an dem oder den Wirbeln anliegen. Die Prothese hat dann diejenige Position zwischen den Wirbeln, die durch den Tiefenabstand zwischen den Wirbel- und Prothesenanschlägen bestimmt ist. Um die Prothese unterschiedlich tief in ventral-dorsaler Richtung einsetzen und unterschiedliche anatomische Verhältnisse berücksichtigen zu können, ist dieser Tiefenabstand zweckmäßigerweise veränderbar. Dies kann beispielsweise dadurch geschehen, daß unterschiedliche Schieber mit unterschiedlichem Tiefenabstand zwischen den Wirbel- und Prothesenanschlägen verfügbar sind, die wahlweise in das Instrument eingesetzt werden. Oder der Schieber ist mit einem der beiden Anschläge ständig fest verbunden, während der andere Anschlag in unterschiedlichen Formaten verfügbar oder in unterschiedlichen Raststellungen mit dem Schieber verbindbar ist. Bevorzugt wird eine Ausführung, bei welcher eine Verstelleinrichtung vorgesehen ist, die zum Verändern des Abstands zwischen dem Prothesenanschlag und dem Wirbelanschlag ausgebildet ist. Vorzugsweise ist sie an dem Schieber angeordnet und weist für einen der beiden Anschläge eine Gleitführung mit Gewindespindel auf. Präoperativ durch Auswertung von Röntgenaufnahmen oder intraoperativ durch Ausmessen kann festgestellt werden, wie groß der Tiefenabstand zwischen der Prothese und der Anschlagkante der Wirbel sein soll. Auf diesen Wert wird dann an der Verstelleinrichtung der Tiefenabstand zwischen den Wirbel- und Prothesenanschlägen des Instruments eingestellt. Zweckmäßigerweise ist sie dazu mit einer Einstellskala versehen. Führt man das Instrument so weit ein, bis seine Wirbelanschläge oder sein Wirbelanschlag die Anschlagkante des zugeordneten Wirbels bzw. der Wirbel erreicht, ist Gewähr dafür vorhanden, daß die Prothese in der gewünschten Tiefe positioniert ist.

Die Erfindung wird im folgenden näher unter Bezugnahme auf die Zeichnung erläutert, die ein vorteilhaftes Ausführungsbeispiel veranschaulicht. Es zeigen:
- Fig. 1: eine Gesamtansicht des Instruments,
- Fig. 2: eine Darstellung des vorderen Instrumentenabschnitts in vergrößertem Maßstab,
- Fig. 3: eine der Fig. 2 entsprechende Darstellung mit angesetzter Abdrückzange,
- Fig. 4: eine der Fig. 3 entsprechende Darstellung in lotrechter Blickrichtung,
- Fig. 5: die Abdrückzange,
- Fig. 6: eine Teilansicht der Abdrückzange in Sichtrichtung A der Fig. 5,
- Fig. 7: eine Einzelheit des Schiebers,
- Fig. 8: die Gesamtansicht eines zweiten Instruments,
- Fig. 9: eine perspektivische Ansicht des vorderen Abschnitts dieses Instruments,
- Fig. 10: eine Seitenansicht des vorderen Abschnitts,
- Fig. 11: eine Unteransicht des vorderen Abschnitts,
- Fig. 12: eine Unteransicht des hinteren Abschnitts und
- Fig. 13: eine Einzelheit der Betätigungseinrichtung.

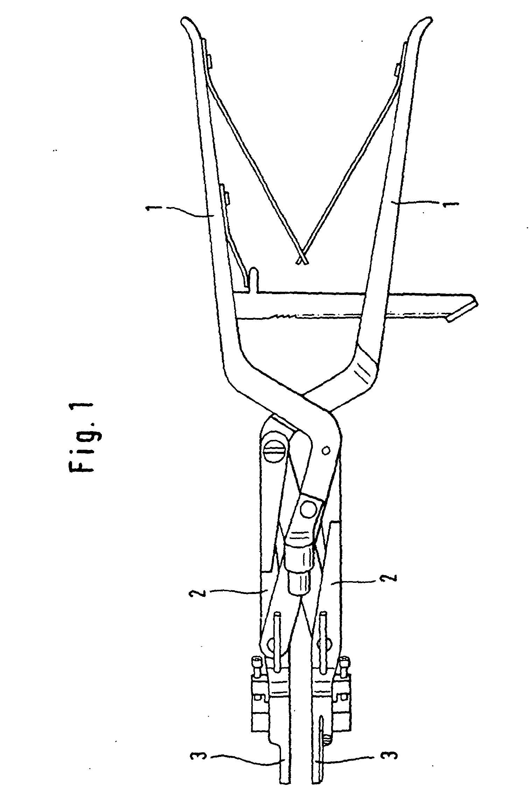

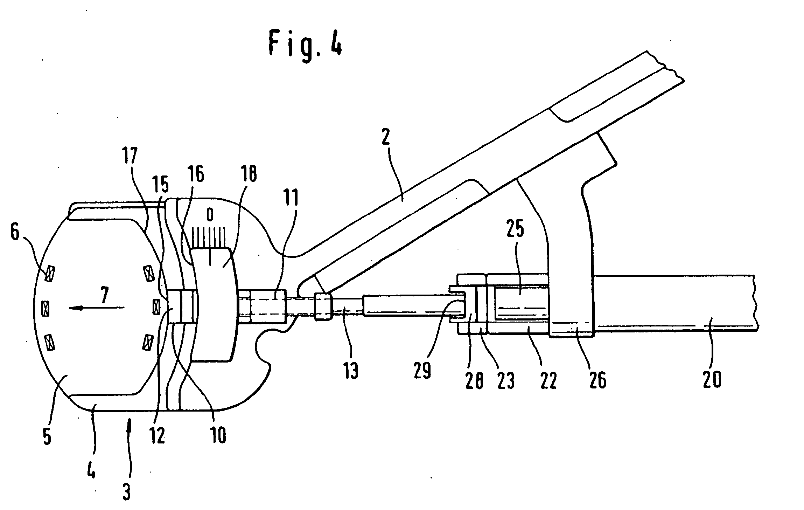

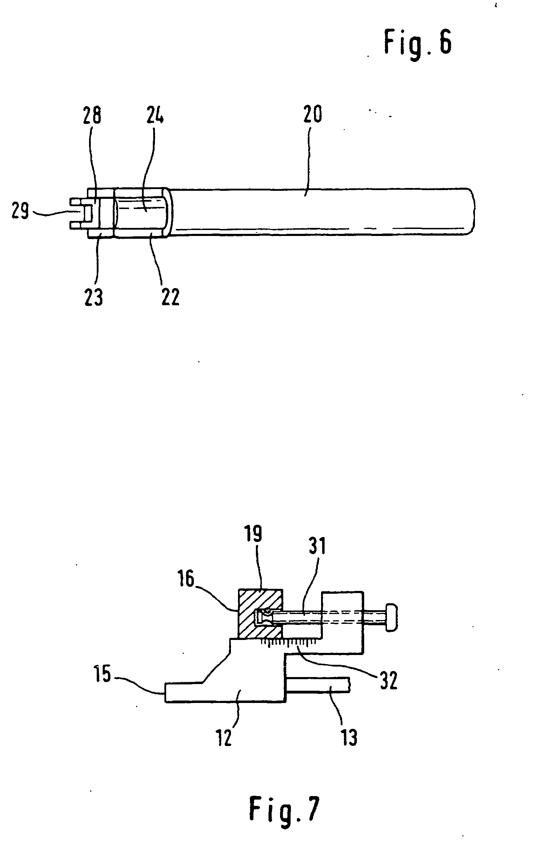

Das in Fig. 1 bis 7 gezeigte Instrument ist eine Spreizzange mit Handhebeln 1 und parallel geführten Arbeitsbügeln 2, die an ihren vorderen Enden je eine Prothesenhalterung 3 tragen. Wie man am besten in Fig. 4 erkennt, sind die Prothesenhalterungen 3 U-förmig mit zwei Seitenschenkeln 4 ausgebildet. Sie nehmen in Gleitnuten die Seitenkanten je einer Prothesenplatte 5 auf. In Fig. 4 blickt man auf die Außenseite einer Prothesenplatte 5, die zur Anlage an einem Wirbelkörper bestimmt ist und Zähne 6 zur besseren Verbindung damit trägt. Die Prothesenhalterung ist so ausgebildet, daß die Prothese hinreichend leicht in Pfeilrichtung 7 herausgezogen werden kann. Gegen unbeabsichtigtes Herausfallen ist sie gesichert durch eine Feder 8. Wegen Einzelheiten der Ausführung und Benutzung kann auf die EP-B-333990 verwiesen werden.

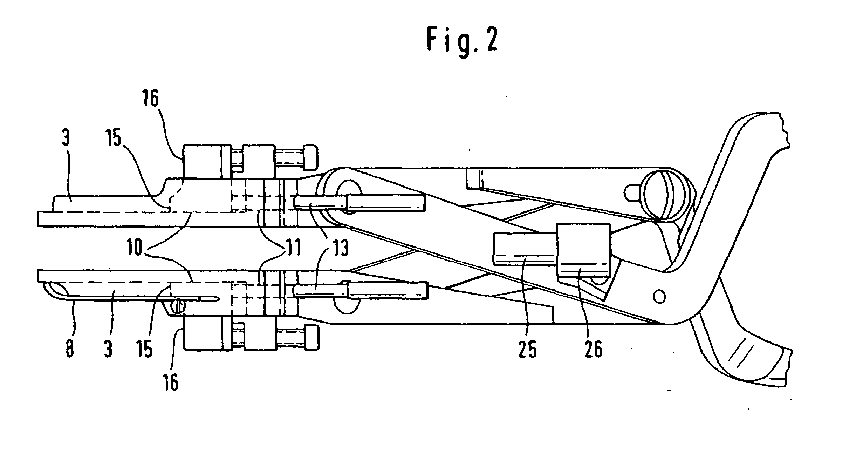

Hinter dem für die Aufnahme der Prothese bestimmten Raum bildet die Prothesenhalterung 3 mittig eine in derselben Richtung angeordnete Führungsnut 10, an die sich nach hinten in gleicher Richtung eine Bohrung 11 anschließt. Die Nut 10 nimmt einen Schieber 12 auf, der darin in Nutrichtung leicht gleitfähig, aber sicher geführt ist und mit einer Stange 13 verbunden ist, die durch die Bohrung 11 und aus dieser hinten herausgeführt ist. Der Schieber 12 kann in Längsrichtung in der Nut 10 verschoben werden, wie sich an der unterschiedlichen Einstellung der unteren und oberen Schieber in Fig. 2 erkennen läßt.

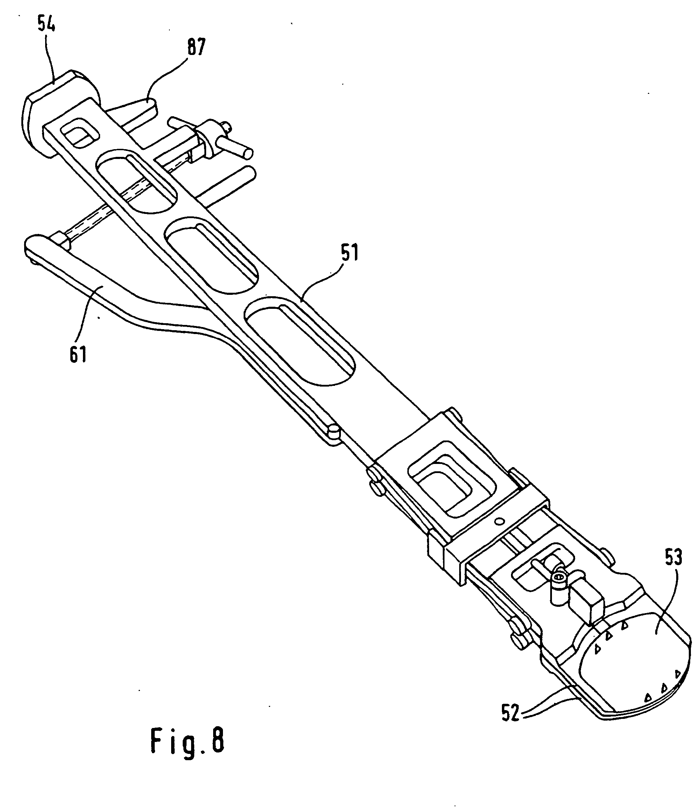

Der Schieber 12 bildet einen Prothesenanschlag 15 und einen gegenüber diesem ein wenig zurückliegenden Wirbelanschlag 16, die im dargestellten Beispiel fest miteinander verbunden sind. Der Prothesenanschlag 15 liegt in der hinteren Endstellung des Schiebers fluchtend mit der Hinterkante 17 des für die Aufnahme der Prothese 5 vorgesehenen Raums. Das Instrument wird so weit in den Wirbelzwischenraum eingeführt, daß der wirbelanschlag am ventralen Rand der betroffenen Wirbel anliegt. Der Abstand zwischen den Anschlägen 15 und 16 bestimmt somit die Einsetztiefe der Prothese. Wenn man den den Wirbelanschlag 16 bildenden Teil 18 auf dem Schieber 12 verstellbar macht, kann man die Einsetztiefe der Prothese variabel vorbestimmen. Die vergrößerte Darstellung in Fig. 7 zeigt einen Schieber 12, an dem der Wirbelanschlag 16 von einem besonderen Klötzchen 19 gebildet ist, das mittels einer Gewindespindel 31 im Verhältnis zum Prothesenanschlag 15 verstellt werden kann. Zur leichteren Einstellung und Kontrolle des Abstands zwischen Prothesenanschlag 15 und Wirbelanschlag 16 ist eine Einstellskala 32 an dem Schieber 12 vorgesehen. In dem dargestellten Beispiel wirkt sie mit einer Hinterkante des Wirbelanschlags 16 als Zeiger zusammen. Skala und Zeiger können auch anders angeordnet sein, zum Beispiel die Strichmarkierung an einer Seitenfläche des Wirbelanschlags 16 und die Skala an einer Oberfläche der Prothesenführung 3.

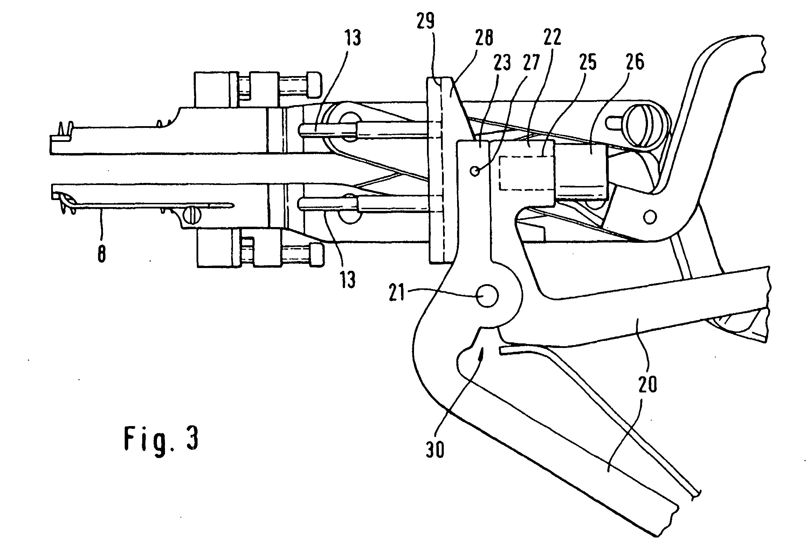

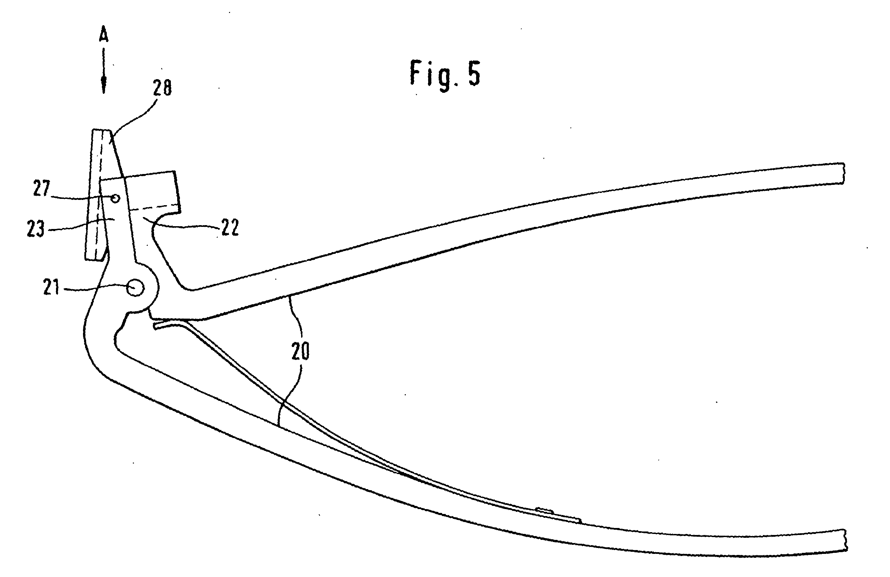

Man erkennt bei der Betrachtung der Fig. 4 ohne weiteres, daß der Prothesenanschlag die Prothese aus der Prothesenhalterung herausschiebt, wenn der Schieber vorgeschoben wird. Wenn die Prothese zwischen zwei Wirbeln festgelegt ist, wird dadurch das Instrument von der Prothese abgedrückt. Dazu muß man (siehe Fig. 2 und 3) die Stangen 13 im Verhältnis zum Instrument nach vorne drücken. Dies geschieht durch die in Fig. 5 gezeigte Abdrückzange, die leicht mit dem Instrument verbunden werden kann. Ihre federgespreizten Griffschenkel 20 gehen jenseits des Gelenkpunkts 21 in Arbeitshebel 22, 23 über, die im wesentlichen quer zur Richtung der Griffschenkel 20 verlaufen. Der Arbeitshebel 22 enthält eine U-förmig umgrenzte Aufnahmeöffnung 24, deren Weite wenig größer ist als der Durchmesser eines Stifts 25 an einer Konsole 26, die derart an dem Instrument angeordnet ist, daß sie unabhängig von der jeweiligen Spreizstellung der Prothesenhalterungen 3 gegenüber den Stangen 13 etwa mittig angeordnet ist.

An dem vorderen Arbeitshebel 23 ist um eine Achse 27 schwenkbar eine Wippe 28 angebracht. Der Schwenkpunkt 27 liegt dann, wenn die Abdrückzange an das Instrument angesetzt ist (Fig. 3) etwa in Richtung der Mittelachse des Stifts 25. Die dem Stift 25 abgewendete Seite der Wippe 28 enthält eine Nut 29, deren Weite ein wenig größer ist als der Durchmesser der Stangen 13 an deren hinteren Enden. Wenn man die Spreizzange in Pfeilrichtung 30 (Fig. 3) an das Instrument ansetzt, so daß der Stift 25 von der Öffnung 24 aufgenommen werden kann, gleiten die hinteren Enden der Stangen 13 in die Nut 29 und liegen dann etwa gleich weit beiderseits des Schwenkpunkts 27 der Wippe 28. Werden nun die Griffschenkel 20 der Abdrückzange zusammengedrückt, bewegt sich der Arbeitshebel 23 im Verhältnis zur Konsole 26, die den Arbeitshebel 22 abstützt, nach vorne, so daß über die Wippe 28 die Stangen 13 und damit die Schieber 12 nach vorne bewegt werden. Dadurch werden die in der Prothesenhalterung befindlichen Prothesenplatten nach vorne aus der Führung herausgedrückt oder - umgekehrt ausgedrückt - wird das Instrument von der Prothese abgedrückt. Ein Teil der Abdrückkraft wird dabei über die Wirbelanschläge 16 auch an den Wirbeln abgetragen. Falls dies unerwünscht ist, so können mittels der Verstelleinrichtung nach dem Positionieren der Prothese und unmittelbar vor dem Abdrücken des Instruments die Wirbelanschläge 16 ein wenig zurückgezogen werden, so daß sie nicht mehr an den Wirbeln anliegen. Die Wirbel bleiben dann beim Abdrücken kraftfrei.

Die Fig. 8 bis 12 zeigen eine Ausführungsalternative. Am vorderen Ende des Instrumentenkörpers 51 befinden sich zwei Halterungen 52 für Prothesenplatten 53. Die Prothesenhalterungen 52 sind gabelförmig und am Ende offen. Ihre seitlichen Schenkel bilden Führungen für den Rand der Prothesenplatten 53. Sie gestatten es, die Prothesenplatten unter Überwindung einer Reibkraft in Längsrichtung des Instruments in die Prothesenhalterungen 52 einzusetzen bzw. herausgleiten zu lassen. Am hinteren Ende besitzt der Prothesenkörper 51 eine Schlagplatte 54. Durch Schläge auf diese Platte können die Prothesenplatten 53, gehalten von den Prothesenhalterungen 52, zwischen zwei Wirbelkörper getrieben werden.

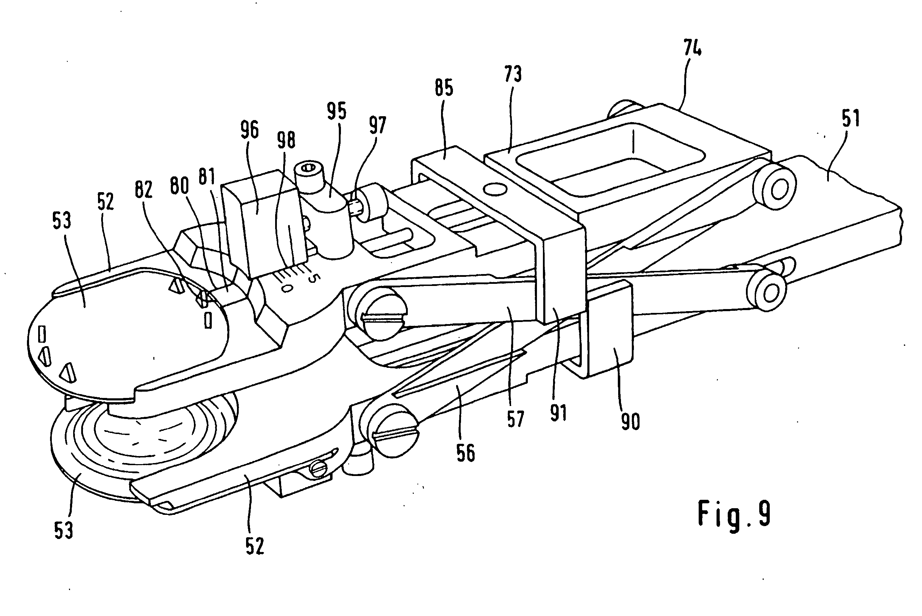

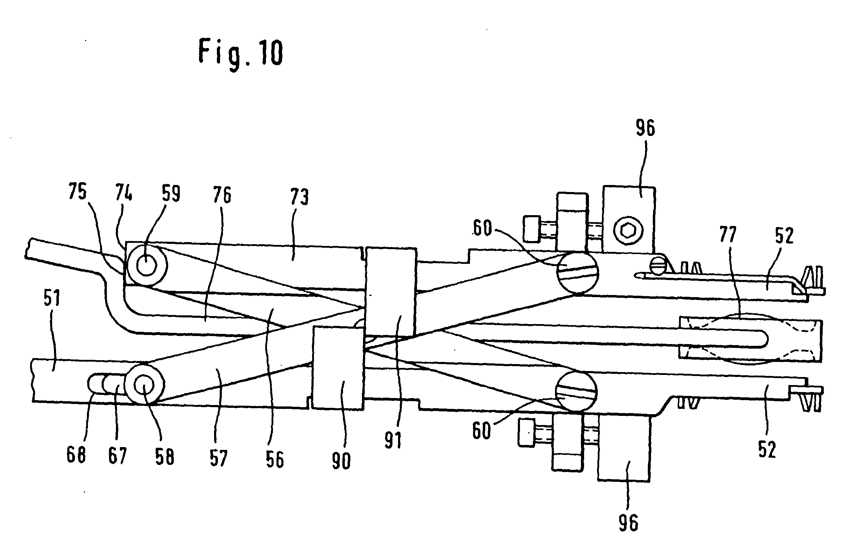

Die untere der Prothesenhalterungen 52 (Fig. 9 und 10) ist mit dem Instrumentenkörper 51 fest und im dargestellten Beispiel sogar einstückig verbunden. Die obere Prothesenhalterung 52 ist mittels einer Scherenanordnung, die aus Scherengliedern. 56, 57 besteht, mit dem Instrumentenkörper 51 verbunden. Die Scherenanordnung 56, 57 ist so gestaltet, daß die obere Prothesenhalterung 52 sich ausschließlich lotrecht zur unteren Prothesenhalterung 52 und parallel zu dieser bewegen kann. Die Prothesenhalterungen können einander weitestmöglich angenähert werden (Fig. 1), um leichter in den Wirbelzwischenraum eingetrieben werden zu können. Sie können zusammen mit den benachbarten Wirbeln auseinandergespreizt werden (Fig. 9 und 10), um Raum zu geben für die Einführung des Prothesenkerns zwischen die Prothesenplatten 53. Danach werden sie einander wieder angenähert, um den Prothesenkern in der gewünschten Stellung festzuhalten. Das Instrument kann dann abgezogen werden.

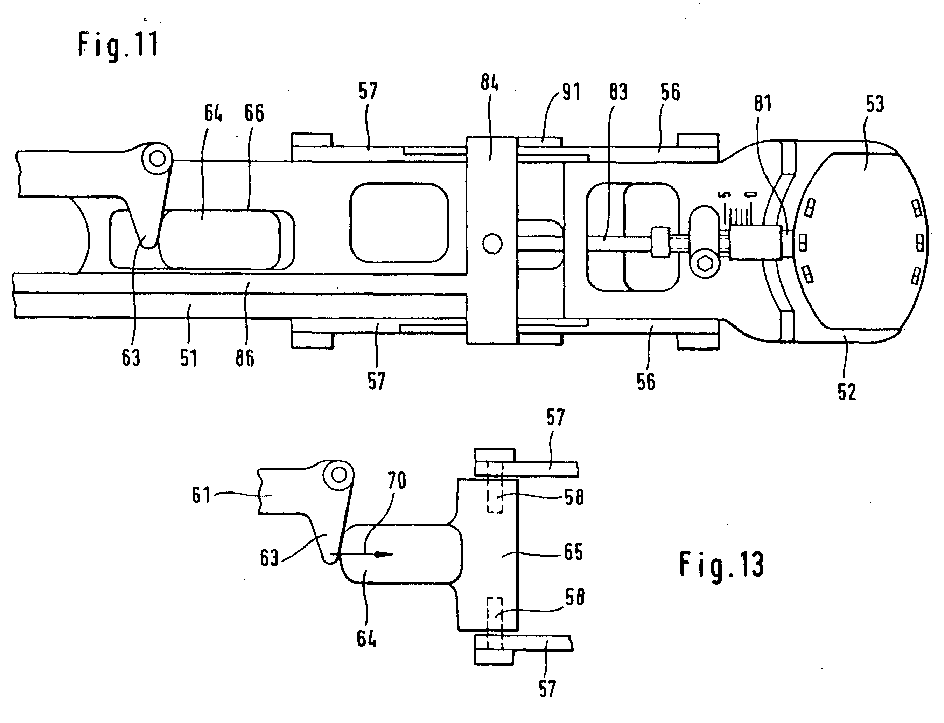

Die hinteren Bolzen 58, 59 der Scherenglieder 56, 57 gleiten in Langlöchern des Instrumentenköpers 51, deren Richtung mit der Instrumentenlängsrichtung übereinstimmt, während die vorderen Bolzen 60 starr mit den Prothesenhalterungen 52 verbunden sind. Um die Prothesenhalterungen zu spreizen, ist eine Einrichtung vorgesehen, die den hinteren Bolzen 58 des Scherenglieds 57 in Längsrichtung des Instruments verschiebt. Dafür ist der Griffhebel 61 vorgesehen, der am Instrumentenkörper um eine Achse 62 schwenkbar ist und einen Arbeitshebel 63 aufweist, der auf das hintere Ende eines Gleitsteins 64 wirkt, der Teil eines T-förmigen Schlittens 65 (Fig. 13) ist, an dessen Querhaupt die hinteren Bolzen 58 der beiderseits angeordneten Scherenglieder 57 angelenkt sind. Der Schlitten 65 ist in Längsrichtung des Werkzeugkörpers geführt. Man erkennt in Fig. 11, daß die parallelen Kanten des Gleitsteins 64 zwischen entsprechend parallelen Kanten 66 eines Ausschnitts im Instrumentenkörper geführt sind. In Fig. 10 erkennt man, daß die Enden 67 des Querhaupts in Langlöchern 68 geführt sind. Wird der Griffhebel 61 wie beim Zusammendrücken der Hebel einer Zange an den Instrumentenkörper herangezogen, so drückt sein Arbeitshebel 63 den Schlitten 64 in Pfeilrichtung 70 (Fig. 13). Dadurch wird das hintere Ende des Scherenglieds 57 nach vorne getrieben, wodurch die Prothesenhalterungen 52 auseinandergespreizt werden. Der Arbeitshebel 63, der Schlitten 65 und die Schräglenker 57 bilden somit eine Anordnung zum Verstellen des Abstands der Prothesenhalterungen 52. Es versteht sich, daß diese Anordnung auch durch andere Ausführungen ersetzt werden kann. Ferner erkennt man, daß die Spreizkraft nicht unbedingt über die Schräglenker 57 ausgeübt zu werden braucht. Falls der Winkel zwischen dem Schräglenker 57 und der Längsrichtung des Instruments zu klein ist für die Ausübung einer großen Spreizkraft, kann für die Spreizung ein gesondertes Glied vorgesehen werden.

Beim Spreizen der Prothesenhalterungen treten beträchtliche Kräfte auf. Deshalb ist der Handhebel 61 durch eine Gewindespindel 71 mit Knebelmutter 72 ergänzt, die den Vorgang erleichtert und es gestattet, das Instrument in der gespreizten Stellung zeitweilig festzusetzen.

In dieser Stellung bildet sich zwischen dem Instrumentenkörper 51 und der die obere Prothesenhalterung 52 nach hinten fortsetzenden Platte 53 einerseits sowie zwischen den seitlichen Scherenanordnungen 56, 57 andererseits ein kanalartiger Freiraum. Durch diesen Freiraum kann mittels eines Instruments 76 der Prothesenkern 77 zwischen die Prothesenplatten 52 geführt werden (Fig. 10). Das Instrument 76 weist einen Anschlag 75 auf, der sich dann an die Hinterkante 74 der Platte 73 anlegt, wenn der Prothesenkern 77 genau die vorgesehene Stellung zwischen den Prothesenplatten 52 erreicht hat.

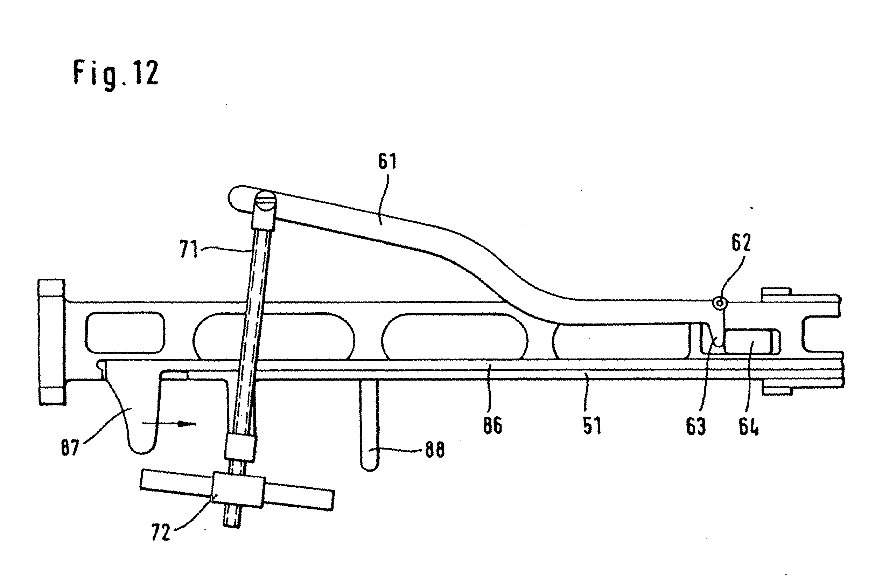

Es wird nun die Einrichtung zum Auswerfen der Prothesenplatten 53 aus den Prothesenhalterungen 52 bzw. zum Abdrücken des Instruments von den Prothesenhalterungen bzw. den angrenzenden wirbeln beschrieben. Die Prothesenführungen 52 enthalten hinter dem Aufnahmeraum für die Prothesenplatten 53 eine Führungsnut 80, die in Instrumentenlängsrichtung und somit in der Gleitrichtung der Prothesenhalterungen 52 verläuft. Sie enthält einen Schieber 81, dessen vorderes Ende 82 am Rand der in der Prothesenhalterung befindlichen Prothesenplatte anschlägt und deswegen als Prothesenanschlag bezeichnet wird. Das hintere, in Fig. 9 und 11 nicht sichtbare Ende des Schiebers 81 ist mit einer ebenfalls in Instrumentenlängsrichtung geführten Stange 83 starr verbunden. Das hintere Ende der im Instrumentenkörper 51 gelagerten Stange 83 ist, wie Fig. 11 zeigt, an einem Anschlagelement 84 befestigt, dessen Natur später erläutert wird. Es ist ebenfalls in Längsrichtung des Instruments verschiebbar. Das Anschlagelement 84 ist wiederum starr mit einer Schubstange 86 verbunden, die im Instrumentenkörper 51 längs verschieblich gelagert ist und (s. Fig. 12) zu einer Handhabe 87 führt. Wenn der Operateur die Handhabe 87 in Pfeilrichtung nach vorne schiebt, werden die Schubstange 86, das Anschlagelement 84, die Stange 83 und der Schieber 81 nach vorne verschoben, um die Prothesenplatte 53 aus der Prothesenhalterung 52 herauszuschieben. Dabei kann sich die Hand des Operateurs an einem Zapfen 88 abstützen (Fig. 12), der fest mit dem Instrumentenkörper 51 verbunden ist.

Unmittelbar wirkt sich die Bewegung der Handhabe 87 nur auf den Schieber 81 aus, der im unteren Teil des Instruments, nämlich im Instrumentenkörper, angeordnet ist. Damit sich die Schieber 81 beider Prothesenhalterungen synchron bewegen, ist eine Bewegungsübertragungseinrichtung vorgesehen. Die den Schieber 81 der oberen Prothesenhalterung steuernde Stange 83 ist an ihrem hinteren Ende mit einem Anschlagelement 85 fest verbunden, das ebenso wie das Anschlagelement 84 der unteren Prothesenhalterung in Instrumentenlängsrichtung beweglich geführt ist. Das untere Anschlagelement 84 weist beiderseits hochragende Anschlagschenkel 90 auf, die hinter und benachbart den Schenkeln 91 liegen, die von dem oberen Anschlagelement 85 beiderseits herunterragen. Wenn die Prothesenplatten 53 sich in ihrer hintersten Stellung in den Prothesenhalterungen 52 befinden und die Prothesenanschläge 82 sie berühren, liegen auch die einander benachbarten Stirnflächen der Anschlagschenkel 90, 91 aneinander an. Wenn nun durch Betätigung der Handhabe 87 das untere Anschlagelement 84 mit den Anschlagschenkeln 90 nach vorne geschoben wird, wird durch deren Zusammenwirken mit den Anschlagschenkeln 91 des oberen Anschlagelements auch der Schieber 81 der oberen Prothesenhalterung nach vorne geschoben. Die beiden Schieber 81 bewegen sich somit synchron. Da die zusammenwirkenden Anschlagflächen 90, 91 lotrecht zur Instrumentenlängsrichtung verlaufen, ist die synchrone Bewegung der Schieber 81 unabhängig von dem jeweiligen Abstand der Prothesenhalterungen voneinander gewährleistet.

Jeder Schieber 81 trägt einen starr mit ihm verbundenen Ansatz 95 sowie ein in Längsrichtung des Schiebers an diesem geführtes Klötzchen 96, das mit seiner Stirnfläche den Wirbelanschlag bildet. Wenn die Prothesenhalterungen mit den darin befindlichen Prothesenplatten 53 in den Zwischenraum zweier Wirbel eingetrieben werden, legen sich die Stirnflächen der Wirbelanschläge 96 schließlich an die ventralen Kanten der Wirbelkörper an. Durch den Abstand der Stirnflächen der Wirbelanschläge 96 von den Prothesenplatten wird somit die Tiefe bestimmt, in der die Prothesenplatten in den Wirbelzwischenraum gelangen. Durch Verstellen der Wirbelanschläge 96 an den Schiebern 81 kann diese Tiefe verändert werden. Dies geschieht mittels einer Gewindespindel 97, die in einer Gewindebohrung des Ansatzes 95 geführt ist und deren Ende drehbar, aber in Längsrichtung fest mit dem Wirbelanschlag 96 verbunden ist. Durch Verdrehen der Gewindespindel 97 kann somit der Operateur die Einsetztiefe der Prothesenplatten 53 im Verhältnis zur ventralen Kante der zugehörigen Wirbelkörper vorherbestimmen. Dabei hilft ihm eine Skala 98.

## Patentansprüche

1. Chirurgisches Instrument zum Einsetzen einer Zwischenwirbel-Endoprothese, die aus zwei Prothesenplatten (5,53) und einem von diesen eingeschlossenen Prothesenkern (77) besteht, das zwei auseinanderspreizbare endoffene Prothesenhalterungen (3, 52) und eine Abdrückeinrichtung zum Abdrücken des Instruments von den in den Prothesenhalterungen (3,52) befindlichen Prothesenplatten (5,53) und/oder mindestens einem der benachbarten Wirbel umfaßt **dadurch gekennzeichnet, daß** die Abdrückeinrictung an jeder Prothesenhalterung (3,52) einen Schieber (12,81) umfaßt, der einen Prothesenanschlag (15,82) und/oder einen Wirbelanschlag (16,96) aufweist und in einer Schieberführung (10,80) zwischen einer zurückgezogenen und einer vorgeschobenen Endstellung mittels einer Betätigungseinrichtung verschiebbar ist, die eine vom gegenseitigen Abstand der Prothesenplatten unabhängige Einrichtung (28,29,90,91) zur Kopplung der Bewegung beider Schieber umfaßt.

2. Instrument nach Anspruch 1, **dadurch gekennzeichnet, daß** eine Einrichtung zum Übersetzen der Antriebskraft zwischen der Abdrückeinrichtung und einer Handhabe (20) vorgesehen ist.

3. Instrument nach Anspruch 2, **dadurch gekennzeichnet, daß** die Übersetzungseinrichtung von einer Hebelanordnung (20 bis 23) gebildet ist.

4. Instrument nach Anspruch 3, **dadurch gekennzeichnet, daß** die Handhabe (20), gegebenenfalls mit der Übersetzungseinrichtung (20 bis 23), mit dem in Operationsstellung befindlichen Instrument leicht verbindbar ist.

5. Instrument nach Anspruch 4, daß die Handhabe (20) und die Übersetzungseinrichtung (20 bis 23) von einer Zange gebildet sind und die Zange an einem ihrer Arbeitshebel (22) und das Instrument komplementäre Kupplungsmittel (24, 25) aufweisen, während der andere Arbeitshebel (23) auf den Schieber (12) wirkt.

6. Instrument nach Anspruch 5, **dadurch gekennzeichnet, daß** zwischen der Abdrückeinrichtung und den damit zusammenwirkenden Teilen (13) der Schieber (12) eine als abstandsunabhängige Einrichtung zur Kopplung in deren Bewegung eine Wippe (28) angeordnet ist.

7. Instrument nach einem der Ansprüche 1 bis 6, **dadurch gekennzeichnet, daß** die Betätigungseinrichtung an einen lediglich mit einer der beiden Prothesenhalterungen (52) fest verbundenen Instrumentenkörper (51) angeordnet ist und der Schieber (81) dieser Prothesenhalterung mit einem Treiberteil (84) bewegungsverbunden ist, der über eine abstandsunabhängige Kupplung (90,91) mit einem getriebenen Teil (85) des anderen Schiebers (81) in Eingriff steht.

8. Instrument nach Anspruch 7, **dadurch gekennzeichnet, daß** der Treiberteil (90) und der getriebene Teil (91) als in Abdrückrichtung geführten Anschläge ausgebildet sind.

9. Instrument nach Anspruch 7 oder 8, **dadurch gekennzeichnet, daß** der mit einer der Prothesenhalterungen (52) fest verbundene Instrumentenkörper (51) eine Schubstange (86) mit Griff (87) enthält, die mit dem zugehörigen Schieber (81) bewegungsverbunden ist.

10. Instrument nach einem der Ansprüche 1 bis 9, **dadurch gekennzeichnet, daß** wenigstens ein Schieber (12,81) einen Prothesenanschlag (15,82) und einen Wirbelanschlag (16,96) aufweist und der Abstand dieser Anschläge voneinander in Tiefenrichtung veränderbar ist.

11. Instrument nach Anspruch 10, **dadurch gekennzeichnet, daß** es eine Verstelleinrichtung zum Verändern des Abstands zwischen dem Prothesenanschlag (15,82) und dem Wirbelanschlag (16,96) aufweist.

12. Instrument nach Anspruch 11, **dadurch gekennzeichnet, daß** die Verstelleinrichtung an dem Schieber (12,81) angeordnet ist und eine Gleitführung mit Gewindespindel (31,97) für einen der beiden Anschläge (16,96) aufweist.

13. Instrument nach Anspruch 11 oder 12, **dadurch gekennzeichnet, daß** die Verstelleinrichtung eine Einstellskala (32,98) aufweist.

14. Instrument nach Anspruch 1 bis 13, **dadurch gekennzeichnet, daß** die kinematischen Verbindungen (56, 57, 90, 91) zwischen den die beiden Prothesenhalterungen (52) tragenden Instrumententeilen (51, 73) außerhalb einer mittleren, in Instrumentenrichtung verlaufenden Durchtrittsöffnung angeordnet sind, deren Weite mindestens der Breite des Prothesenkerns (77) und eines Einbringinstruments (76) dafür entspricht.

## Claims

1. A surgical instrument for the insertion of an intervertebral endoprosthesis which consists of two prosthesis plates (5,53) and a prosthesis core (77), which is enclosed by them and which comprises two prosthesis holders (3,52) open at the end and able to be spread apart, and an ejection device for ejecting the instrument from the prosthesis plates (5,53) situated in the prosthesis holders (3,52) and/or from at least one of the adjacent vertebrae, **characterised in that** the ejection device on each prosthesis holder (3,52) comprises a slider (12,81) which has a prosthesis abutment (15,82) and/or a vertebral abutment (16,96) and is displaceable in a slider guide (10,80) between a retracted and an advanced end position by means of an actuating device which comprises a device (28,29,90,91), which is independent of the mutual spacing of the prosthesis plates, for coupling the movement of both sliders.

2. An instrument according to Claim 1, **characterised in that** a device is provided for transmitting the drive force between the ejection device and a manipulator (20).

3. An instrument according to Claim 2, **characterised in that** the transmission device is formed by a lever arrangement (20 to 23).

4. An instrument according to Claim 3, **characterised in that** the manipulator (20), optionally with the transmission device (20 to 23), can be readily connected to the instrument disposed in the operating position.

5. An instrument according to Claim 4, **characterised in that** the manipulator (20) and the transmission device (20 to 23) are formed by forceps and the forceps have on one of their operating levers (22) coupling means (24,25) complementary to the instrument, whereas the other operating lever (23) acts on the slider (12).

6. An instrument according to Claim 5, **characterised in that**, between the ejection device and the parts (13) of the slider (12) co-operating therewith, a rocker (28) is disposed as a device which is not distance-dependent for coupling them in their movement.

7. An instrument according to any one of Claims 1 to 6, **characterised in that** the actuating device is disposed on an instrument body (51) securely connected only to one of the two prosthesis holders (52) and the slider (81) of this prosthesis holder is connected for movement with a drive member (84) which is in engagement with a driven member (85) of the other slider (81) via a coupling (90,91) which is not distance-dependent.

8. An instrument according to Claim 7, **characterised in that** the drive member (90) and the driven member (91) are in the form of stop members guided in the ejection direction.

9. An instrument according to Claim 7 or 8, **characterised in that** the instrument body (51) securely connected to one of the prosthesis holders (52) includes a push rod (86) with a handle (87) which is connected for movement with the associated slider (81).

10. An instrument according to any one of Claims 1 to 9, **characterised in that** at least one slider (12,81) has a prosthesis abutment (15,82) and a vertebral abutment (16,96), and the distance apart of these abutments is variable in depth direction.

11. An instrument according to Claim 10, **characterised in that** it has an adjusting device for varying the distance between the prosthesis abutment (15,82) and a vertebral abutment (16,96).

12. An instrument according to Claim 11, **characterised in that** the adjusting device is disposed on the slider (12,81) and has a sliding guide with a threaded spindle (31,97) for one of the two abutments (16,96).

13. An instrument according to Claim 11 or 12, **characterised in that** the adjusting device has a control scale (32, 98).

14. An instrument according to Claims 1 to 13, **characterised in that** the kinematic connections (56,57,90,91) between the instrument parts (51,73) carrying the two prosthesis holders (52) are disposed outside a central passage opening extending in the direction of the instrument, the width of which opening corresponds at least to the width of the prosthesis core (77) and an insertion instrument (76) therefor.

## Revendications

1. Instrument chirurgical pour insérer une endoprothèse intravertébrale qui est constituée de deux plaques de prothèse (5, 53) et d'un noyau de prothèse (77) enfermé par celles-ci, qui comprend deux fixations de prothèse (3, 52) ouvertes à leur extrémité et pouvant être écartées l'une de l'autre ainsi qu'un dispositif de pression pour repousser l'instrument des plaques de prothèse (5, 53) se trouvant dans les fixations de prothèse (3, 52) et/ou au moins de l'une des vertèbres voisines, **caractérisé en ce que** le dispositif de pression comprend sur chaque fixation de prothèse (3, 52) un coulisseau (12, 81) qui comporte une butée de prothèse (15, 82) et/ou une butée de vertèbre (16, 96), et peut coulisser dans un guide de coulisseau (10, 80) entre une position de fin de course reculée et une position de fin de course avancée, au moyen d'un dispositif d'actionnement qui comprend un dispositif (28, 29, 90, 91) indépendant de l'écartement des plaques de prothèse pour le couplage du mouvement des deux coulisseaux.

2. Instrument selon la revendication 1, **caractérisé en ce qu'**un dispositif est prévu pour transmettre la force d'entraînement entre le dispositif de pression et une poignée (20).

3. Instrument selon la revendication 2, **caractérisé en ce que** le dispositif de transmission est formé par un dispositif à leviers (20 à 23).

4. Instrument selon la revendication 3, **caractérisé en ce que** la poignée (20), éventuellement avec le dispositif de transmission (20 à 23), peut être facilement reliée à l'instrument se trouvant en position d'opération.

5. Instrument selon la revendication 4, **caractérisé en ce que** la poignée (20) et le dispositif de transmission (20 à 23) sont formés par une pince et la pince, sur l'un de ses leviers de travail (22), ainsi que l'instrument comportent des moyens d'accouplement (24, 25) complémentaires, tandis que l'autre levier de travail (23) agit sur le coulisseau (12).

6. Instrument selon la revendication 5, **caractérisé en ce qu'**entre le dispositif de pression et les parties (13), coopérant avec celui-ci, des coulisseaux (12), est disposée une bascule (28) servant de dispositif indépendant de l'écartement, pour le couplage dans leur mouvement.

7. Instrument selon l'une des revendications 1 à 6, **caractérisé en ce que** le dispositif d'actionnement est disposé sur un corps d'instrument (51) relié de manière fixe uniquement à l'une des deux fixations de prothèse (52), et le coulisseau (81) de cette fixation de prothèse est relié en mouvement à une partie d'entraînement (84) qui est en prise, par un accouplement (90, 91) indépendant de l'écartement, avec une partie menée (85) de l'autre coulisseau (81).

8. Instrument selon la revendication 7, **caractérisé en ce que** la partie d'entraînement (90) et la partie menée (91) sont réalisées en tant que butées guidées dans le sens de la pression.

9. Instrument selon la revendication 7 ou 8, **caractérisé en ce que** le corps d'instrument (51), relié de manière fixe à l'une des fixations de prothèse (52), contient une bielle (86) avec poignée (87) qui est reliée en mouvement au coulisseau (81) associé.

10. Instrument selon l'une des revendications 1 à 9, **caractérisé en ce qu'**au moins un coulisseau (12, 81) comporte une butée de prothèse (15, 82) et une butée de vertèbre (16, 96), et l'écartement de ces butées peut être modifié dans le sens de la profondeur.

11. Instrument selon la revendication 10, **caractérisé en ce qu'**il comporte un dispositif de réglage pour modifier l'écartement entre la butée de prothèse (15, 82) et la butée de vertèbre (16, 96).

12. Instrument selon la revendication 11, **caractérisé en ce que** le dispositif de réglage est disposé sur le coulisseau (12, 81) et comporte un guide à glissement avec broche filetée (31, 97) pour l'une des deux butées (16, 96).

13. Instrument selon la revendication 11 ou 12, **caractérisé en ce que** le dispositif de réglage présente une échelle de réglage (32, 98).

14. Instrument selon les revendications 1 à 13, **caractérisé en ce que** les liaisons cinématiques (56, 57, 90, 91) entre les parties de l'instrument (51, 73) portant les deux fixations de prothèse (52), sont disposées à l'extérieur d'une ouverture de passage central, s'étendant dans la direction de l'instrument, dont la largeur correspond pour cela au moins à la largeur du noyau de prothèse (77) et d'un instrument d'introduction (76).
